# EUROPEAN PATENT APPLICATION

(11) **EP 3 996 398 A1**
(43) Date of publication of application: **11.05.2022**
(21) Application number: 21153266.8
(22) Date of filing: 25.01.2021
(51) Int. Cl.: H04W 4/80

(54) **METHOD, DEVICE AND SYSTEM FOR CONNECTING A MEDICAL DEVICE TO A NETWORK**

(30) Priority: 05.11.2020 WO PCT/CN2020/126689
(71) Applicant: Koninklijke Philips N.V., 5656 AG Eindhoven (NL)
(72) Inventor: ZHOU, Xiao-Ming, 5656 AE Eindhoven (NL)
(74) Representative: Philips Intellectual Property & Standards

(57) **Abstract**

A method for connecting a medical device to a network is provided. The method comprises receiving, from a server of the network or via a user interface, a pairing request by a guiding device; sending, from the guiding device to a pairing device independent from the medical device, a pairing message including the configuration information for connecting the medical device to the network; configuring the pairing device based on the configuration information to enable the pairing device to receive and interpret a broadcast message from the medical device while the guiding device waits for, from the pairing device, a pairing acknowledge (indicating a success or a failure of pairing the medical device with the pairing device; sending, from the pairing device to the guiding device, the pairing acknowledge indicating the success of pairing the medical device with the pairing device if the broadcast message of the medical device can be received and interpreted and the pairing acknowledge indicating the failure of pairing the medical device with the pairing device if the broadcast message of the medical device cannot be received and interpreted. By doing so, the medical device may be commissioned to a network flexibly and scalably.

## Description

### ▪FIELD OF THE INVENTION

The present invention generally relates to a medical field, especially relates to connecting a medical device to a network.

### BACKGROUND OF THE INVENTION

It is well known that an AED (Automated External Defibrillator) is typically a standalone device and placed in a public space as a first-aid lifesaver. AEDs of some brands may routinely broadcast its device data, for instance battery level, to enable the maintenance of the AEDs. Nowadays, connecting AED to a network, as AED IoT solution becomes a trend that makes AED locating, maintenance and management much easier. Due to the high requirement for use safety and/or frequently changed scenario of applications, AED device provides only one-way data communication, e.g., broadcast message. Currently there is no convenient method to connect a standalone AED automatically to a network.

The procedure of connecting a device to a network is called commissioning. To enable AED IoT networking, some manufacturers may pre-save network configuration such as an IP address in a chip of AED device when it is in the product line. However, in this approach the wireless protocol and IP address for AED commission must be fixed and the whole system becomes inflexible after delivery.

At present, the conventional way of connecting an AED to a network requires human interventions, for instance, a system installer first connects an AED to the so-called AED case embedded with different sensors and then connects the AED case to a server via different wireless networks. The system installer needs to secure the AED case can receive the broadcast message from the AED and the AED case can be connected to the wireless network for data communication, for instance, to transmit the data received from the broadcast message to remote server of the network. When wireless signal is weak or human errors occur during the network configuration, extra workflows are needed to test all functions work properly. This makes AED IoT networking lack of scalability.

### SUMMARY OF THE INVENTION

It would be desirable to commission a medical device provided by different manufacturers to a network in a flexible and scalable way with reduced human interventions.

The inventor realized that AEDs from different manufacturers may adopt different wireless protocols for message broadcasting, when an AED case is bound to an AED device with specific configuration and capable of commissioning to a specific AED IoT network, it may be incapable of commissioning another AED device with a different configuration to the network due to the lack of compatibility of wireless communication protocol used by AEDs from different manufacturers.

The inventor further realized that an AED IoT system installer who facilitates commissioning an AED to the AED IoT network often confronts a connectivity issue during installation due to weak wireless signal or temporary network failures, in such situation there will be no real-time feedback available.

The general idea of this invention is to introduce a pairing device and a guiding device to commission an AED device to a network and based on the technical solution proposed by this invention, there is no need to pre-save configuration information needed for commissioning an AED device during production in a plant or to bind an AED case to an AED device from a specific manufacturer. Instead, the pairing device is configured dynamically according to the configuration information received from the guiding device and adapted to latest configuration under a surveillance of the guiding device.

The commissioning of the AED device includes two pairing steps, e.g., a pairing between the AED device and the pairing device and a pairing between the pairing device and the server of the network. By configuring a pairing device dynamically with the aid of a guiding device, the pairing device can receive broadcast messages from AED devices using different communication protocols and send the same to a remote server having a specific network address. Further, the guiding device is introduced not only to assist in the configuration of the pairing device, but also provide an opportunity to the pairing device to obtain a real-time feedback from the remote server through the guiding device during installation.

The discussion of commissioning an AED device to a network is applicable to any medical device, which has similar nature of use safety and/or frequently changed scenario of applications and provides only one-way communication, for instance, broadcast message.

In one aspect, the present invention provides a method for connecting a medical device to a network and executed in a pairing device independent from the medical device, the method comprises
receiving, from a guiding device, a pairing message including configuration information for connecting the medical device to the network; configuring the pairing device based on the configuration information to enable the pairing device to receive and interpret a broadcast message from the medical device; and
sending, to the guiding device, a pairing acknowledge indicating a success of pairing the medical device with the pairing device if the broadcast message of the medical device can be received and interpreted.

Since the pairing device is dynamicallly configurable based on the configuration information received from the guiding device, the pairing device may be used for commissioning an AED device from a different manufacturer to a network. There is no need to pre-save the configuration information during production in a plant or to bind an AED case to an AED device from a specific manufacturer.

In another aspect, the present invention provides a method for connecting a medical device to a network and executed in a portable guiding device, the method comprises receiving, from a server of the network or from a user interface, a pairing request; sending, to a pairing device, a pairing message including a configuration information for connecting the medical device to the network to enable the pairing device to sense and interpret the broadcast message from the medical device; waiting for, from the pairing device, a pairing acknowledge indicating a success or a failure of pairing the medical device with the pairing device; and sending, to the server or to the user interface, a paring notification indicating the success or the failure of the pairing the medical device with the pairing device.

With above operations of the guiding device, it enables dynamically configuring the pairing device based on the sent configuration information and to provide real time feedbacks on the commission to the installer.

In a further aspect, the present invention provides a method for connecting a medical device to a network, the method comprises receiving, from a server of the network or via a user interface, a pairing request by a guiding device; sending, from the guiding device to a pairing device independent from the medical device, a pairing message including the configuration information for connecting the medical device to the network; configuring the pairing device based on the configuration information to enable the pairing device to receive and interpret a broadcast message from the medical device while the guiding device waits for, from the pairing device, a pairing acknowledge indicating a success or a failure of pairing the medical device with the pairing device; sending, from the pairing device to the guiding device, the pairing acknowledge indicating the success of pairing the medical device with the pairing device if the broadcast message of the medical device can be received and interpreted and the pairing acknowledge indicating the failure of pairing the medical device with the pairing device if the broadcast message of the medical device cannot be received and interpreted; and sending, from the guiding device to the server or the user interface, a pairing notification indicating the success or the failure of the pairing the medical device with the pairing device.

According to above method, the guiding device sends configuration information to a pairing device in response to a pairing request and the pairing device is dynamically configured with the configuration information received from the guiding device and returns feedbacks to the guiding device. By doing so, the pairing device is configured dynamically with the aid of the guiding device and is capable of commissioning any AED devices with different communication protocols.

In a further aspect, the present invention provides a pairing device for connecting a medical device to a network, the pairing device being independent from the medical device, the pairing device comprises a receiving unit for receiving, from a guiding device, a pairing message including configuration information for connecting the medical device to the network; a controlling unit for configuring the pairing device based on the configuration information to enable the pairing device to receive and interpret a broadcast message from the medical device; and a sending unit for sending, to the guiding device, a pairing acknowledge indicating a success of pairing the medical device with the pairing device if the broadcast message of the medical device can be received and interpreted.

In a further aspect, the present invention provides a portable guiding device for connecting a medical device to a network, the portable guiding device comprises
a receiving unit for receiving a pairing request, from a server of the network or from a user interface; a sending unit for sending, to a pairing device, a pairing message including the configuration information for connecting the medical device to the network to enable the pairing device to sense and interpret the broadcast message from the medical device; and
a controlling unit for controlling the receiving unit to wait for, from the pairing device, a pairing acknowledge indicating the success or failure of pairing the medical device with the pairing device and controlling the sending unit to send, to the server or to the user interface, a paring notification indicating the success of the pairing the medical device with the pairing device.

In a further aspect, the present invention provides a system for connecting a medical device to a network comprising the portable guiding device and the pairing device of the embodiments of the present invention.

In a still further aspect, the present invention provides a computer readable medium storing a computer program, when executed by a processor or a computer, for performing the method of the embodiments of the present invention.

According to an embodiment of above aspects, the configuration information includes a product model of the medical device, the method comprises
mapping, based on a predefined configuration table, the product model of the medical device to a specific wireless communication protocol for sensing and interpreting the broadcast message from the medical device.

In this embodiment, by setting the predefined configuration table as long as the product model of the AED device is known, the pairing device may configure itself conveniently. The product model refers to any information of the AED device that can solely correspond to or represent the wireless communication protocol of the AED device, it includes but is not limiting to, its brand name, a name or a number of its product series, or corresponding data structure.

For example, different brands of medical devices can have different product models, as long as the predefined configuration table stores a brand name of the AED device and its corresponding wireless communication protocol, with the brand name of the AED device received from the guiding device, the pairing device may configure itself based on its corresponding wireless communication protocol stored in the configuration table. This facilitates the installer to use the pairing device to commission the AED device. This reduces the skill requirements for the installer to commission the AED device.

According to an embodiment of above aspects, the configuration information includes a product model and wireless communication protocol, the method further comprises adding the configuration information to a predefined configuration table if the configuration information is not included in the predefined configuration table yet.

For this, the predefined configuration table can be updated in response to receiving a new product model and its corresponding wireless communication protocol. Therefore, the pairing device may be used for further commissioning medical devices with the new product model in the future. It provides scalability for the pairing device.

According to an embodiment of above aspects, the method further comprises
determining if the pairing device stores a driver corresponding to the configuration information of the medical device; and configuring the pairing device with the driver corresponding to the configuration information of the medical device to receive the broadcast message from the medical device if it is determined that the pairing device stores the driver.

By configuring the pairing device with a suitable driver for the AED device, the pairing device may receive the broadcast message from the AED device.

According to an embodiment of above aspects, the method further comprises
determining if the broadcast message from the medical device can be interpreted based on a predefined translation table stored in the paring device; and generating the pairing acknowledge indicating the success of pairing the medical device with the pairing device if it is determined that the broadcast message from the medical device can be interpreted based on the predefined translation table.
By doing so, the pairing device is paired with the AED device to receive and interpret the broadcast message. And further a feedback may be generated to indicate the success of pairing the AED device with the pairing device, therefore, an installer may obtain the feedback when the pairing succeeds.

According to an embodiment of above aspects, the pairing device stores at least one driver and/or at least one predefined translation table that are updatable respectively based on updating information from the guiding device and/or the server.

By storing at least one driver, the pairing device is not limited to be paired with only a specific AED device to receive the broadcast message therefrom, but it has the ability to be paired with a different AED device with different configuration information from a different manufacturer.

For example, when the pairing device stores drivers for a Bluetooth communication protocol and a Zigbee communication protocol respectively, the pairing device may be configured with a respective driver to receive the broadcast messages from an AED device with the Bluetooth communication protocol and/or receive the broadcast messages from another AED device with the Zigbee communication protocol.

By storing at least one predefined translation table, the broadcast messages from at least one AED device from different manufacturers can be interpreted respectively by the pairing device.

By updating the at least one driver and/or at least one predefined translation table, the pairing device may be scalable to be used for commissioning different AED devices that were originally un-supported by the pairing device.

According to a further embodiment of above aspects, the configuration information includes networking settings, the method further comprises
sending a networking request to a server of the network based on the networking settings; waiting for a networking acknowledge from the server and/or the guiding device;
repeating the sending and waiting steps with a predefined interval within a predefined period before the networking acknowledge is received; and
sending, to the guiding device, a notification indicating a success of connecting the medical device to the network if the networking acknowledge is received from the server or a notification indicating the failure of connecting the medical device to the network if no networking acknowledge is received from the server or the guiding device.

By doing so, a standalone AED device is automatically connected to a network and then the broadcast message may be sent to the server. With different networking settings, the pairing device may be connected to different networks such that the AED device may be connected to different networks via the pairing device. By sending a feedback to the guiding device, the installer may understand if the commissioning the AED device succeeds timely.

Various aspects and features of the disclosure are described in further detail below. And other objects and advantages of the present invention will become more apparent and will be easily understood with reference to the description made in combination with the accompanying drawings.

### DESCRIPTION OF THE DRAWINGS

The present invention will be described and explained hereinafter in more detail in combination with embodiments and with reference to the drawings, wherein:
Fig. 1 shows a scenario connecting each AED device to a network according to one embodiment of the present invention.
Fig. 2 is a flow chart of a method for connecting the AED device to a network according to one embodiment of the present invention.
Fig. 3 is a flow chart of a method for connecting the AED device to the network according to another embodiment of the present invention.
Fig. 4 is a flow chart of a method for updating a predefined translation table in a pairing device according to one embodiment of the present invention.
Fig. 5 shows a system for connecting the AED device to the network according to one embodiment of the present invention.
Fig. 6 shows a system for connecting the AED device to the network according to another embodiment of the present invention.

The same reference signs in the figures indicate similar or corresponding feature and/or functionality.

The present invention will be described with respect to particular embodiments and with reference to certain drawings, but the invention is not limited thereto. The drawings described are only schematic and are non-limiting. In the drawings, the size of some of the elements may be exaggerated and not drawn on scale for illustrative purposes.

### DETAILED DESCRIPTION OF THE INVENTION

The embodiments of the present invention will be described below with reference to an automated external defibrillator (AED), however, it is not limiting and the embodiments of the present invention may be applied to any other medical devices also.

With respect to the wording "broadcast message", it shall be understood that it does not limit the communication manner of the medical device to the broadcast, it only refers to message sent from the medical devices. The communication manner of the medical device can also include groupcast, unicast and so on.

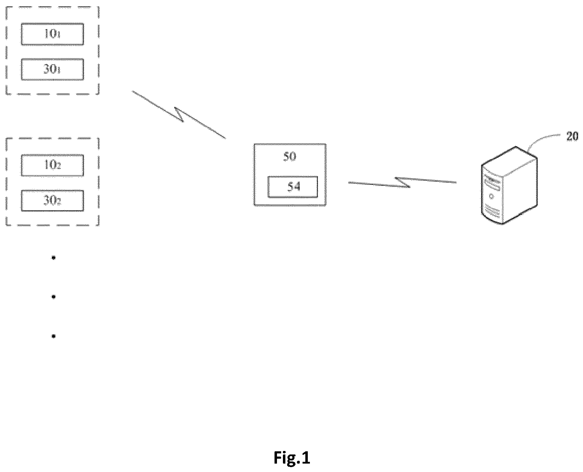

Fig. 1 shows a scenario connecting each AED device to a network according to one embodiment of the present invention. A plurality of AED devices 10₁, 10₂ may be placed at different locations in a public space to be used for emergency. Each AED device can be located in a corresponding case in the public space and the corresponding case can include a pairing device for connecting the AED device to the network. As shown in Fig. 1, the AED device 10₁ and the pairing device 30₁ are located in one case and the AED device 10₂ and the pairing device 30₂ are located in another case.

After use, the AED devices 10₁, 10₂ are respectively placed in corresponding cases to provide one-way communication, messages broadcasted from the AED devices 10₁, 10₂ are desired to be sent to a remote server 20 via corresponding pairing devices 30₁, 30₂. By introducing the pairing device, commissioning the AED device to the network relates to pairing the AED device to the pairing device and then pairing the pairing device to the server. Above both pairings are performed under the assistance of a portable guiding device 50. In particular, the guiding device 50 may be used for guiding and monitoring above pairings. The guiding device 50 can include a user interface 54 for receiving and/or outputting information to a user, therefore, the feedbacks during installation and communication can be delivered via the guiding device 50.

The guiding device 50 is a portable device that can be carried by a user. Especially, the guiding device 50 can be any kinds of smart devices that can provide communications with other devices. For example, the guiding device is a mobile phone or a tablet computer and so on. Or the guiding device can be embodied as an application that can be installed and run in a smart device.

Although as shown in Fig. 1, the guiding device 50 is in communication with the pairing device 30₁ to assist in connecting the AED device 10₁ to the server 20, it is illustrative but not limiting, the guiding device 50 can also be in communication with the pairing device 30₂ to assist in connecting the AED device 10₂ to the server 20, even the guiding device 50 can be in communication with both pairing devices 30₁, 30₂ to assist in connecting the AED devices 10₁ and 10₂ to the server 20.

Further, although as shown in Fig. 1, only one pairing device is included in one case for connecting a corresponding AED device to the server 20, it is only illustrative but not limiting. It can be contemplated that multiple medical devices can share a same pairing device and be provided in a case together with the pairing device. In one example, the multiple AED devices can broadcast messages based on different wireless protocols, and the pairing device can be configured to receive the messages from each of the multiple AED devices based on a different wireless communication protocol. For example, one AED device broadcasts the message based on Bluetooth, and the other AED device broadcasts the message based on infrared, then, the pairing device can receive the message from the one AED device based on Bluetooth and receive the message from the other AED device based on infrared. Thereby, the pairing device may receive the message from different AED devices. In another example, the multiple AED devices may use a same wireless communication protocol, and the pairing device may receive the message from one AED device in one time slot but receive the message from another AED device in a different time slot. Although it is described with respect to the multiple AED devices, the multiple medical devices of different types are also contemplated. The wording "pair" in the specification shall be understood in a broad sense, but not be limited to one to one relation.

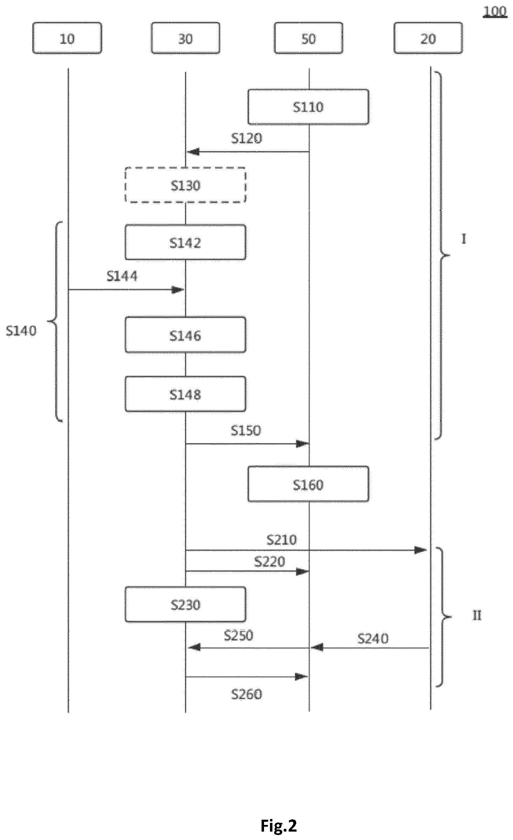

Fig. 2 is a flow chart of a method 100 for connecting an AED device 10 to a network, i.e., commissioning the AED device 10, according to one embodiment of the present invention. The AED device 10 is connected to a server 20 via a pairing device 30 with the assistance of a guiding device 50 to send a message broadcasted from the AED device 10 to the server 20. The method executed in the pairing device 30 and the method executed in the guiding device 50 both are described with reference to the method 100 as shown in Fig. 2.

The method 100 as shown in Fig. 2 relates to a procedure I for pairing the AED device 10 with the pairing device 30 and a procedure II for pairing the pairing device 30 with the server 20. Before the method 100 as shown in Fig. 2 is performed, the guiding device 50 has passed authentication to the server 20 and thus is able to communicate with the server 20, and both the pairing device 30 and the guiding device 50 have to enter a commissioning mode to connect to each other. In one embodiment, the commissioning mode is triggered by clicking a button on the pairing device and clicking a function button of the guiding device.

### Procedure I

In step S110, a pairing request may be received by the guiding device 50, for example via a user interface 54 of the guiding device 50. The pairing request can comprise at least configuration information for connecting the AED device 10 to the network.

In one embodiment, the configuration information includes a wireless communication protocol used by the AED device 10 for broadcasting a message. The wireless communication protocol includes infrared, Zigbee, IP, 6LoWPAN, or Bluetooth.

In another embodiment, the configuration information includes a product model of the AED device 10 received via the user interface 54. The product model of the AED device 10 can be any information that solely identifies the communication protocol of the AED device. It includes, but is not limiting to, its brand name, a name or a number of its product series, or corresponding data structure.

In a still another embodiment, the configuration information includes both the product model of the AED device 10 and corresponding wireless communication protocol.

After in the step S110 the pairing request including the configuration information is received, a pairing message including the configuration information is generated and sent to the pairing device 30 by the guiding device 50 in a step S120. Accordingly, the pairing device 30 receives the pairing message including the configuration information from the guiding device 50 in the step S120.

In one embodiment, during the step 120, if the pairing device 30 cannot receive the pairing message from the guiding device 50 within a predefined time interval after their connection, the pairing device 30 may send a feedback to the guiding device 50 to ensure if the guiding device 50 has sent the pairing message; or if the pairing device 30 receives the pairing message from the guiding device 50 within the predefined time interval, it can also send a feedback to the guiding device 50 to give a feedback on the receiving of the pairing message.

The pairing device 30 can store a predefined configuration table in which each product model of the AED device is stored in correspondence to a specific wireless communication protocol. when the product model of the AED device 10 is received by the pairing device 30 from the guiding device 50 in the step S120, the method can proceed to a step S130 in which the product model of the AED device 10 received from the guiding device 50 is mapped by the pairing device 30 to the specific wireless communication protocol based on the predefined configuration table. After determining the wireless communication protocol, the method proceeds to a step S140.

If the received configuration information includes both the product model of the AED device and corresponding wireless communication protocol, the pairing device 30 may first determine if the product model of the AED device and the corresponding wireless communication protocol is stored in the predefined configuration table, if they are not stored, it can be contemplated that the predefined configuration table may be updated based on the received product model and wireless communication protocol by the pairing device 30.

In this case, in the step S130, the product model of the AED device and corresponding wireless communication protocol of the received configuration information may be added to the predefined configuration table for facilitating a next time use. After updating the predefined configuration table, the method proceeds to the step S140.

Although the step S130 is described with reference to the pairing device 30 as above, it is also contemplated that the step S130 is performed in the guiding device 50, that is to say, the guiding device 50 stores the predefined configuration table, maps the configuration information to the specific wireless communication protocol based on the predefined configuration table, and, if necessary, updates the information in the predefined configuration table. After that, the specific wireless communication protocol is sent from the guiding device 50 to the pairing device 30.

When the configuration information received by the pairing device 30 includes the wireless communication protocol of the AED device 10 but not the product model of the AED device 10, the step S130 may be omitted and the method proceeds to the step S140 directly.

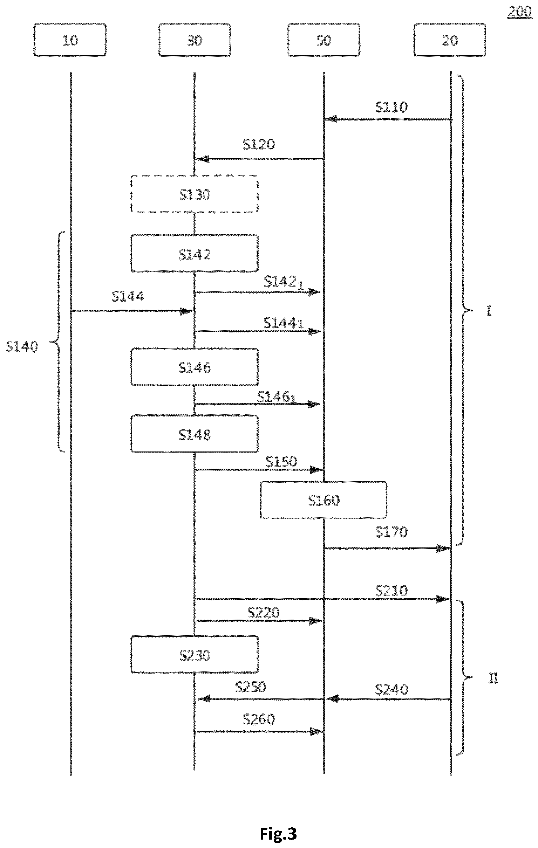

In the step S140, the pairing device 30 configures itself, especially its receiving unit, based on the configuration information to enable the pairing device 30 to receive and interpret a broadcast message from the AED device 10. Meanwhile, the guiding device 50 waits for a pairing acknowledge indicating a success or a failure of pairing the AED device 10 with the pairing device 30. The guiding device 50 can also receive various feedbacks from the pairing device 30 during the step S140 and display the feedbacks to a user, such that the user can easily understand where the configuration goes and/or where goes wrong. The feedbacks will be described with reference to Fig. 3 in detail.

The step S140 further comprises steps S142-S148. In the step S142, the pairing device 30 determines if the configuration information of the AED device 10 can be supported by the pairing device 30, i.e., if the pairing device 30 stores a driver corresponding to the configuration information of the AED device 10.

It is contemplated that the pairing device 30 stores at least one driver each corresponding to a different configuration information of an AED device.

When the configuration information includes a wireless communication protocol of the AED device, the pairing device 30 can store different drivers each corresponding to a different wireless communication protocol for the AED devices. When the configuration information includes a product model of the medical device that can solely identify the wireless communication protocol of the medical device, the pairing device 30 can store different drivers each corresponding to a different product model so as to configure the pairing device 30 with a suitable driver corresponding to the received product model from the guiding device 50. If the pairing device 30 stores different drivers each corresponding to a different product model, there is no need to map the product model of the medical device to a specific wireless communication protocol, therefore, the step S130 may be omitted also.

When the pairing device 30 obtains the wireless communication protocol or the product model of the AED device 10, it determines if a driver corresponding to the wireless communication protocol or the product model is stored in the pairing device 30, thereby determining if the configuration information of the AED device 10 may be supported by the pairing device 30. If it is determined that a corresponding driver is stored, the pairing device 30 configures its receiving unit with the corresponding driver in the step S142 to facilitate receiving the broadcast message from the AED device 10. Otherwise, the pairing device 30 records that the configuration information cannot be supported by the pairing device 30 and the method can proceed to a step S148.

In an example, if the pairing device 30 recognizes that a product type of the medical device uses an infrared communication protocol, it will determine if a driver corresponding to the infrared communication protocol is stored and configures the receiving unit with the driver corresponding to the infrared communication protocol if it is determined that the driver is stored.

When the receiving unit of the pairing device 30 has been configured with a driver corresponding to the AED device 10 in the step S142, the method proceeds to a step S144. In the step S144, the pairing device 30 detects a broadcast messages from the AED device 10, if the pairing device 30 can detect and receive the broadcast message from the AED device 10 within a predefined time interval in the step S144, the method proceeds to a step S146. Otherwise, the pairing device 30 records that the pairing device 30 cannot detect the broadcast message from the AED device 10 and the method can proceed to a step S148.

In the step S146, after the broadcast message has been received from the AED device 10, the pairing device 30 further determines if the received broadcast message can be translated into readable data, that is to say, if the pairing device 30 can interpret the received broadcast message. Generally, the pairing device 30 can store at least one predefined translation table which defines how to interpret the broadcast message from the AED devices. Each translation table includes translation data and may be used to interpret the broadcast message of one data type. If a predefined translation table corresponding to the data type of the broadcast message of current AED device has been stored in the pairing device 30, it can be determined that the pairing device 30 supports the interpretation of the broadcast message from the current AED device. Further, if the predefined translation table stores translation data for the broadcast message, it can be determined that the broadcast message from the medical device may be interpreted based on the predefined translation table.

For example, if the pairing device 30 stores a predefined translation table corresponding to a data type such as binary of an AED device 10 of a brand, it means that the pairing device supports the interpretation of the broadcast message from the AED device 10 of the brand, but the broadcast message from a AED device of a different brand with a different data type cannot be interpreted.

The broadcast message can be interpreted based on the translation data stored in the predefined translation table. For example, if the translation data shows that every 4 bits are used as a cell for the broadcast message of the AED device 10, first 4 bits with 0000 refer to a battery level of the AED device 10 and second 4 bits with 0001 refer to a current battery level of 85%, when a broadcast message "0000 0001" is received from the AED device 10 in the step S144, the broadcast message can be interpreted as "a current battery level is 85%" in the step S146.

After the broadcast message is interpreted to the readable data, the readable data may be stored in a memory of the pairing device 30 in the step S146 for further transmitting to the server 20 after pairing the pairing device 30 with the server 20. Otherwise, the pairing device 30 records that the broadcast message cannot be interpreted to the readable data and the method can proceed to a step S148.

Further in the step S148, the pairing device 30 generates a pairing acknowledge indicating a success or a failure of pairing the AED device 10 with the pairing device 30 based on the processing results of the steps S142, S144 and/or S146.

If it is determined that the configuration information of the AED device 10 can be supported by the pairing device 30 in the step S142, the broadcast message of the AED device 10 can be received by the pairing device 30 in the step S144 and the broadcast message of the AED device 10 can be interpreted to the readable data in the step S146, that is to say, the broadcast message of the AED device 10 can be received and interpreted by the pairing device 30, a pairing acknowledge indicating a success of pairing the AED device 10 with the pairing device 30 may be generated, such as "AED device paring succeeds".

Otherwise, if any one of the steps S142, S144 and S146 gives negative determination results, a pairing acknowledge indicating a failure of pairing the AED device 10 with the pairing device 30 may be generated.

Please note that if it is determined that the configuration information of the AED device 10 cannot be supported by the pairing device 30 in the step S142, the steps S144, S146 may be omitted, the method proceeds to the step S148 directly to generate a pairing acknowledge indicating a failure of pairing the AED device 10 with the pairing device 30. In addition, if it is determined that the broadcast message of the AED device 10 cannot be received within a predefined time interval in the step S144, the step S146 may be omitted, the method proceeds to the step S148 to generate a pairing acknowledge indicating a failure of pairing the AED device 10 with the pairing device 30 accordingly.

Furthermore, detailed description on the failure of the pairing may be generated. For example, if it is determined in the step S142 that the pairing device 30 does not store a driver for a Bluetooth communication protocol of the AED device 10 and thus the configuration information of the AED device 10 cannot be supported by the pairing device 30, the pairing acknowledge may be generated as "Not supported"; if it is determined that the configuration information of the AED device 10 can be supported by the pairing device 30 in the step S142 but the broadcast message cannot be received from the AED device 10 in the step S144, the pairing acknowledge may be generated as "supported but no broadcast message received"; if it is determined that the configuration information of the AED device 10 can be supported by the pairing device 30 in the step S142, the broadcast message can be received from the AED device 10 in the step S144, but the broadcast message of the AED device 10 cannot be interpreted to the readable data in the step S146, the pairing acknowledge may be generated as "interpreting fail".

After the pairing acknowledge is generated by the pairing device in the step S148, the pairing acknowledge can be sent to the guiding device 50 in a step S150. In particular, if the broadcast message of the AED device 10 can be received and interpreted, a pairing acknowledge indicating the success of pairing the AED device 10 with the pairing device 30 will be received by the guiding device 50 in the step S150. Otherwise, if the broadcast message of the AED device 10 cannot be received and interpreted, a pairing acknowledge indicating the failure of pairing the AED device 10 with the pairing device 30 will be received by the guiding device 50 in the step S150.

After the pairing acknowledge is received by the guiding device 50, a paring notification indicating the success or the failure of the pairing the AED device 10 with the pairing device 30 may be generated based on the pairing acknowledge by the guiding device 50 and sent back to the user interface to display in a step S160. The pairing acknowledge can be displayed to the installer as feedbacks such that he/she may understand whether the pairing between the pairing device 30 and the AED device 10 is ok or not and where goes wrong. Thereby, a procedure I is finished. The procedure I may be performed repeatedly until the pairing device 30 is successfully paired with the AED device 10.

### Procedure II

In steps S210, the pairing device 30 sends a networking request to a server 20 of a network based on networking settings, meanwhile, in a step S220, the pairing device 30 sends a message to the guiding device 50 to inform the guiding device 50 that a networking request has been sent from the pairing device 30 to the server 20. With the step S220, the installer may be aware of the progress of the networking operation of the pairing device 30 via the guiding device 50. The step S220 may be omitted also. The networking settings may be received in the step S110 as a part of the configuration information. The networking settings may be received via the user interface or from the server 20 by the guiding device 50 and generally include the network address of the server 20 of the network. The network address includes but is not limited to the IP address, Wi-Fi address and/or Bluetooth address of the server 20.

After the networking request is sent to the server 20 in the step S210, the pairing device 30 waits for a networking acknowledge within a predefined interval in a step S230. If the server 20 receives the networking request in the step S210, and a networking acknowledge can be generated and sent from the server 20 to the guiding device 50 in a step S240 and then forwarded to the pairing device 30 in a step S250 within the predefined interval. Once the pairing device 30 receives the networking acknowledge within the predefined interval, a notification indicating a success of connecting the medical device 10 to the network can be sent to the guiding device 50 in a step S260. In this case, the installer may be informed that the pairing of the pairing device 30 with the server 20 is ok via the guiding device 50.

Certainly, the steps S250 and S260 may be omitted also since in the step S240 the guiding device 50 has been informed of the networking acknowledge.

Different from the steps S240 and S250 where the networking acknowledge is sent to the guiding device 50 and then forwarded to the pairing device 30, the networking acknowledge may be sent to the pairing device 30 directly. In this case, the step S260 is expected to inform the installer of the networking acknowledge.

If the server 20 cannot receive the networking request in the step S210, the pairing device 30 will return to the step S210 in the step S230 and repeat the steps S210 and S220 for a predefined period until the networking acknowledge is received via the steps S240 and S250. If the networking acknowledge is received within the predefined period via the steps S240 and S250, the pairing device 30 still sends to the guiding device 50 a notification indicating a success of connecting the medical device 10 to the network in the step S260.

Otherwise, if the networking acknowledge is not received within the predefined period, the pairing device 30 will sends to the guiding device 50 a notification indicating a failure of connecting the medical device to the network in the step S260 and further the pairing device 30 may request the guiding device 50 to resend the networking settings for the server 20. The notification may be shown via the user interface 54 of the guiding device 50 to the installer. If a pairing request is received from the server 20, it is also possible that the notification is sent or forwarded to the server 20.

Although the procedure I is described with reference to the step S110-S160 and the procedure II is described with reference to the step S210-S260, it can be contemplated that one or more steps thereof may be omitted/altered/combined to achieve different functions.

Fig. 3 is a flow chart of a method 200 for connecting the AED device to the network according to another embodiment of the present invention.

Different from the method 100 for connecting the AED device 10 to the network as shown in Fig. 2, in the step S110 of the method 200, the pairing request including the configuration information of the AED device 10 is received by the guiding device 50 from the server 20. After receiving the pairing request, a pairing message including the configuration information may be generated by the guiding device 50 and sent to the pairing device 30 in the step S120.

Accordingly, after a paring notification indicating the success or the failure of the pairing the AED device 10 with the pairing device 30 is generated by the guiding device 50 in the step S160, the pairing notification can be sent back to the server 20 in a step S170.

In addition, after each step of the steps S142, S144, and S146, a feedback signal can be sent to the guiding device 50 in steps S142₁, S144₁, and S146₁ respectively, therefore, a user can easily understand the progress of the pairing the AED device 10 with the pairing device 30 and where goes wrong. The feedback signal can be generated by the pairing device 30 respectively in the steps S142, S144 and S146 based on respective operations therein.

For example, if it is determined that the configuration information of the AED device 10 cannot be supported by the pairing device 30 in the step S142, that is, the pairing device 30 does not store a driver for the wireless communication protocol included in the configuration information, the feedback signal can be generated as "Not supported". If it is determined that the configuration information of the AED device 10 can be supported by the pairing device 30 and the receiving unit of the pairing device 30 has been successfully configured with the corresponding driver in the step S142, the feedback signal can be generated as "configuration ok" and sent to the guiding device 50 in the step S142₁. Then, the method 200 proceeds to the step S144 to receive a broadcast message from the AED device 10.

If no broadcast message is received from the AED device 10 within a predefined time slot in the step S144, the feedback signal can be generated as "No broadcast message received"; if there is a broadcast message received from the AED device 10 in the step S144, the feedback signal can be generated as "Broadcast message received", then the feedback signal can be sent to the guiding device 50 in the step S144₁. Then, the method proceeds to the step S146.

If it is determined that the broadcast message from the AED device 10 cannot be interpreted to the readable data in the step S146, that is, no corresponding predefined translation table is stored in the pairing device 30, the feedback signal may be generated as "interpreting fail". If it is determined that the broadcast message from the AED device 10 can be interpreted to the readable data and the broadcast message has been successfully interpreted by the pairing device 30 in the step S146, the feedback signal may be generated as "interpreting ok" and sent to the guiding device 50 in the step S146₁. Then, the method 200 proceeds to the step S148. In the step S148, a pairing acknowledge indicating a success or a failure of pairing the AED device 10 with the pairing device 30 is generated based on the feedback signal from the step S146.

Above feedback signal in the steps S142₁, S144₁, and S146₁ from respective steps S142, S144 and S146 can be shown to the user via the user interface 54 of the guiding device 50. With the feedback signals, the installer may clearly understand where the pairing between the AED device 10 and the pairing device 30 goes and where goes wrong.

Further, according to one embodiment, the driver stored in the pairing device 30 is updatable based on updating information from the guiding device 50 and/or the server 20. In this case, the procedure I can further include the steps of updating the driver and/or the predefined translation table stored in the pairing device 30.

In one example, as shown in Fig. 3, after a feedback signal "not supported" is generated by the pairing device 30, the pairing device 30 not only sends the feedback signal to the guiding device 50 in the step S142₁ but also sends an updating request for the drivers to the guiding device 50. After receiving the updating request from the pairing device 30, the guiding device 50 may provide updating information to the pairing device 30. Thereby, an on-air updating may be provided.
the updating information may include a driver for a wireless communication protocol of the AED device 10 that may not be stored in the pairing device 30 originally. The pairing device 30 receives and stores the driver included in the updating information, and further configures a receiving unit of the pairing device 30 with the driver. Updating the driver in the pairing device 30 relates to adding a new driver to the pairing device or updating one of the drivers stored in the pairing device to a new version.

In another example, the updating of the driver may also be requested by the guiding device 50. For example, if the installer wishes to scale up the pairing device 30 to be paired with an AED device with a new wireless communication protocol, a driver updating request including the updating information for the new wireless communication protocol may be sent from the guiding device 50 to the pairing device 30. The driver updating request may be received by the guiding device 50 from the server and/or the user interface of the guiding device. In response to the updating request from the guiding device 50, the pairing device 30 can update the driver based on the updating information for the new wireless communication protocol and then return an updating acknowledge to the guiding device 50.

In a further example, if the connection between the pairing device 30 and the server 20 has been established, the updating request for the driver may even be sent to the pairing device 30 from the server 20 to obtain the updating information from the server 20.

The pairing device 30 can store not only the drivers corresponding to different configuration information (e.g. the wireless communication protocols) of AED devices of different brands, but also a predefined driver table in which shows each stored driver and its corresponding configuration information.

For example, the paring device 30 can store a driver D1 for infrared communication protocol and a driver D2 for Bluetooth communication protocol, the predefined driver table may show the drivers D1 and D2 and respective communication protocols. Based on the predefined driver table, it may be easily determined whether a suitable driver is stored in the pairing device 30 to correspond to the communication protocol of the AED device 10 in the step S142.

In this case, it is also possible that the updating information includes the driver to be updated and/or added as well as an indicator indicating which kind of the configuration information can be supported by the driver. Then when the driver stored in the pairing device 30 is updated based on the updating information, the predefined driver table may be updated also.

Similar to the driver, the predefined translation table stored in the pairing device 30 can be updated also. The updating of the predefined translation table relates to not only adding, modifying and/or deleting translation data from the predefined translation table but also adding and/or deleting the predefined translation table from the stored predefined translation tables in the pairing device 30. The updating may be requested by the pairing device 30 or the guiding device 50, or if a connection has been established between the pairing device 30 and the server 20 during the procedure II, the updating may be requested by the server 20.

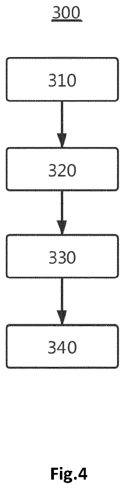

The updating of the translation table may be performed during the procedure I. for example, if it is determined in the step S146 that the pairing device 30 does not store the translation table or the translation data corresponding to the broadcast message received from the AED device 10, the pairing device 30 sends not only a feedback signal "interpreting fail" to the guiding device 50 in the step S146₁, but also an translation table updating request to the guiding device 50. In response to the updating request, the guiding device 50 may sent updating information for the translation table to the pairing device to update the translation table. The updating of the translation table may be initiated by the guiding device 50 or the server 20 also. Fig. 4 is a flow chart of a method 300 for updating the predefined translation table in the pairing device 30 according to one embodiment of the present invention.

In a step S310, both the pairing device 30 and the guiding device 50 enter into the commission mode. This step may be omitted. In a step S320, the guiding device 50 sends a translation table updating request including the updating information to the pairing device 30, the updating request may be received by the guiding device 50 from a user via the user interface or from the server 20. For example, the updating information can indicate to add data such as "first 4 bits with 0001 refers to AED lifecycle and second 4 bits 0010 refer to current AED lifecycle is 50%" into the predefined translation table.

In a step S330, after receiving the translation table updating request, the pairing device 30 updates the predefined translation tables based on the updating information included in the updating request. For example, a predefined translation table may be added with the bits "0001 0010" and its corresponding meaning from above mentioned updating information.

In a step S340, an updating acknowledge can be sent from the pairing device 30 to the guiding device 50 after performing above update.

It can also be contemplated that if the connection between the pairing device 30 and the server 20 has been established, the pairing device 30 may also sends the updating request to the server 20 to obtain the updating information for the translation tables from the server 20, or the server 20 sends the updating request including updating information for the translation tables to the pairing device 30 to initiate the updating.

Although Fig. 4 is shown with reference to updating the translation table, it may also be applied to update the driver.

With the driver and/or the translation table updatable, the pairing device 30 is scalable for an AED device from different manufacturer.

In addition, after above procedures I and II are performed to ensure that the AED device 10 is connected to the server 20 of the network, a test flow may be performed to test if the medical device 10 is connected to the server 20 correctly.

After receiving the notification indicating the success of connecting the AED device 10 to the server 20 of the network in the step S260, the guiding device 50 can further receive a test request via its user interface to initiate the test flow. The guiding device 50 can forward the test request to the pairing device 30. When the pairing device 30 receives the test request, it starts to receive a broadcast message from the AED device 10, once received, it interprets the broadcast message, add a "test" tag to the interpreted message and sends the interpreted message to the server 20, if the server 20 receives the interpreted message with the "test" tag, it can send a feedback indicating a succeed of the test to the guiding device 50, if no such interpreted message is received by the server 20 within a predefined time interval, the server 20 can send a feedback indicating a failure of the test to the guiding device 50.

The pairing device 30 may also send feedbacks to the guiding device 50 during the test flow, for example, when the broadcast message cannot be received from the AED device 10 and/or when the broadcast message cannot be interpreted. With the feedbacks, the user may ascertain what problems occur, thereby facilitating the maintenance of the connection of the AED device 10 to the server 20.

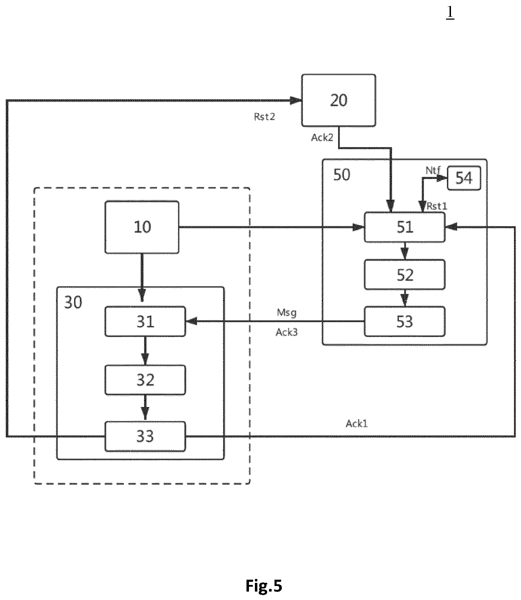

Fig. 5 shows a system 1 for connecting a medical device 10 to a server 20 of a network according to one embodiment of the present invention. The system 1 at least includes a pairing device 30 and a guiding device 50 and optionally includes a medical device such as an AED device 10 and a server 20 of a network.

As shown in Fig. 5, the pairing device 30 comprises a receiving unit 31, a controlling unit 32 and a sending unit 33; the guiding device 50 also comprises a receiving unit 51, a controlling unit 52 and a sending unit 53. Optionally, the guiding device 50 comprises a user interface 54.

The receiving unit 51 receives a pairing request Rst1 from the user interface 54, as described with reference to above step S110, in one embodiment, the configuration information for connecting the AED device 10 to the network may be received as a part of the pairing request Rst1. The configuration information can include a product model of the AED device 10 and/or a wireless communication protocol of the AED device 10. Further, the configuration information can include networking settings for the server 20.

In another embodiment, the pairing request doesn't include the configuration information, but in response to receiving the pairing request, the controlling unit 52 of the guiding device 50 may control the guiding device 50 to obtain corresponding configuration information from the AED device 10, the user interface 54 and/or the server 20 respectively.

Once the configuration information is obtained, a pairing message Msg may be generated to include the configuration information by the controlling unit 52.

The sending unit 53 of the guiding device 50 sends the pairing message Msg to the pairing device 30. The receiving unit 31 of the pairing device 30 receives the pairing message Msg. The controlling unit 32 of the pairing device 30 configures the pairing device 30, especially the receiving unit 31 of the pairing device 30, based on the configuration information included in the pairing message Msg such that the broadcast message from the AED device 10 may be received and interpreted. This corresponds to above step S140. Since the pairing device 30 can be dynamically configured based on the received configuration information from the guiding device, there is no need to pre-save the configuration information during production in a plant or to bind to an AED device from a specific manufacturer. Instead, the pairing device 30 may be dynamically paired with an AED device from any manufacturer to receive the broadcast information from the AED device.

During the configuration of the pairing device 30 by the controlling unit 32 of the pairing device 30, the guiding device 50 waits for a pairing acknowledge Ack1 from the pairing device 30 to indicate a success or a failure of pairing the AED device with the pairing device 30. In particular, the controlling unit 52 of the guiding device 50 controls the receiving unit 51 to wait for, from the pairing device 30, the pairing acknowledge Ack1 indicating the success or the failure of pairing the AED device 10 with the pairing device 30.

If the controlling unit 32 of the pairing device 30 has configured the pairing device 30 to receive and interpret the broadcast message from the AED device 10, the pairing acknowledge Ack1 indicating the success of pairing the AED device 10 with the pairing device 30 is generated by the controlling unit 32. Otherwise, the pairing acknowledge Ack1 indicating the failure of pairing the AED device 10 with the pairing device 30 is generated by the controlling unit 32.

The sending unit 33 of the pairing device 30 sends the pairing acknowledge Ack1 to the guiding device 50. The receiving unit 51 of the guiding device 30 receives the pairing acknowledge Ack1 from the pairing device 30. Thereafter, a pairing notification Ntf indicating the success or the failure of pairing the AED device 10 with the pairing device 30 can be generated based on the received pairing acknowledge Ack1. The pairing notification Ntf may be sent to the user interface 54 from which the pairing request Rst1 is received. Therefore, the procedure I for pairing the AED device 10 with the pairing device 30 is finished.

After the procedure I, the pairing device 30 may be further paired with the server 20 of the network in the procedure II.

During the procedure II, the pairing device 30 sends a networking request Rst2 to the server 20 based on networking settings for the server 20. The networking settings may be included in the configuration information received during the procedure I.

If the server 20 receives the networking request Rst2, it can generate and send a networking acknowledge Ack2 to the guiding device 50. The networking acknowledge Ack2 indicates that a networking request Rst2 from the pairing device 30 has been received based on the networking settings by the server 20. In response to receiving the networking acknowledge Ack2, the guiding device 50 generates a networking acknowledge Ack3 indicating a success of connecting the AED devcie 10 to the network based on the networking acknowledge Ack2.

In one embodiment, the networking acknowledge Ack3 may be sent to the pairing devcie 30, if the networking acknowledge Ack3 is received by the pairing devcie 30, a notification indicating a success of connecitng the AED device 10 to the network may be generated. Otherwise, if the networking acknowledge Ack3 is not received by the pairing devcie 30 within the predefined period, a notification indicating a failure of connecitng the AED device 10 to the network may be generated. The notification can be sent back to the guiding device 50.

In this embodiment, after the networking request Rst2 is sent to the server 10, the controlling unit 32 of the pairing device 30 determines if a networking acknowledge Ack3 is received by the pairing device 30 within a predefined interval from the server 20 and/or the guiding device 50. If not received, the pairing devcie 30 will repeat sending the networking request Rst2 and determining if the networking acknowledge Ack3 is received, until a predefined period expires. If the networking acknowledge Ack3 is received by the pairing device 30 within the predefined period, a notification indicating a success of connecitng the AED device 10 to the network may be generated and sent to the guiding device 50 for display via the user interface.

In another embodiment, the networking acknowledge Ack3 may be omitted. Once the guiding devcie 50 recieves the networking acknowledge Ack2 within the predefined period its controlling unit 52 generates the notification indicating a success of connecitng the AED device 10 to the network to display to the installer. Otherwise, if no the networking acknowledge Ack2 is received within the predefined period, the controlling unit 52 generates the notification indicating the failure of connecting the AED device 10 to the network.

After receiving or generating the notification indicating the success or the failure of connecting the AED device 10 to the network, the guiding device 50 displays the notification to the user via the user interface 54.

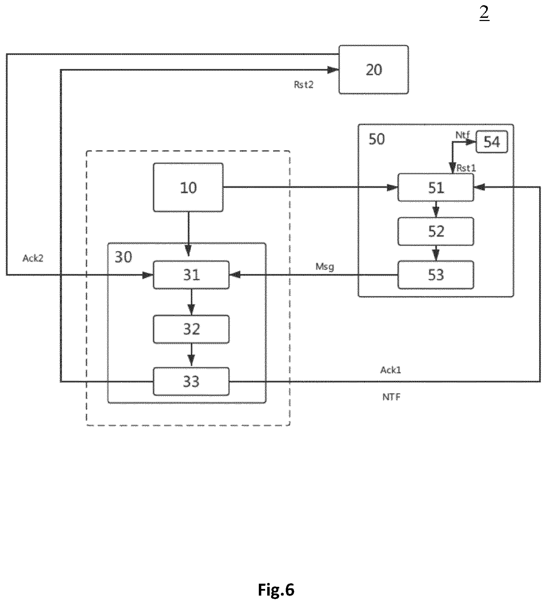

Fig. 6 shows a system 2 for connecting a medical device 10 to a server 20 of a network according to another embodiment of the present invention.

Different from the system 1 as shown in Fig. 5, after the server 20 receives the networking request Rst2 it generates and sends the networking acknowledge Ack2 to the pairing device 30 directly. The pairing device 30 generates a notification NTF indicating a success of connecting the AED device 10 to the network and sent the same to the guiding device 50 for display via the user interface 54.

Although as shown in Figs.5 and 6, the pairing request Rst1 is received from the user interface, it is also contemplated to receive it from the server.

Although the embodiments of the invention are described with reference to different subjects, such as methods and products, the contents described with reference to the different subjects may be combined to each other. In addition, it can be contemplated that the controlling units 32, 52 can performed all controlling or processing functions of the pairing device 30 and the guiding device 50.

The embodiments of the present invention described above may be performed by digital electronic circuitry, in computer software or firmware, in computer hardware, and any combination thereof. In one embodiment, the guiding device may be achieved by one or more memories and one or more processors. The memories stores instructions that are operable, when executed by the one or more processor, to cause the one or more processor to perform the methods of embodiments.

The embodiments of the present invention may be performed by a computer storage medium. The storage medium soring instructions for, when executed by one or more processors, performing the methods of embodiments.

It should be noted that the above-mentioned embodiments illustrate rather than limit the invention and that those skilled in the art would be able to design alternative embodiments without departing from the scope of the appended claims. In the claims, any reference signs placed between parentheses shall not be construed as limiting the claim. The word "comprising" does not exclude the presence of elements or steps not listed in a claim or in the description. The word "a" or "an" preceding an element does not exclude the presence of a plurality of such elements. In the product claims enumerating several units, several of these units can be embodied by one and the same item of software and/or hardware. The usage of the words first, second and third, et cetera, does not indicate any ordering. These words are to be interpreted as names.

## Claims

1. A method (100) for connecting a medical device (10) to a network and executed in a pairing device (30) independent from the medical device, the method (100) comprising:
receiving (S120), from a guiding device (50), a pairing message (Msg) including configuration information for connecting the medical device (10) to the network;
configuring (S140) the pairing device (30) based on the configuration information to enable the pairing device (30) to receive and interpret a broadcast message from the medical device (10); and
sending (S150), to the guiding device (50), a pairing acknowledge (Ackl) indicating a success of pairing the medical device (10) with the pairing device (30) if the broadcast message of the medical device (10) can be received and interpreted.

2. The method (100) of claim 1, wherein the configuration information includes a product model of the medical device (10), the method (100) further comprising:
mapping (S130), based on a predefined configuration table, the product model of the medical device (10) to a specific wireless communication protocol for sensing and interpreting the broadcast message from the medical device (10).

3. The method (100) of claim 1, wherein the configuration information includes a product model and wireless communication protocol, the method (100) further comprising adding (S130) the configuration information to a predefined configuration table if the configuration information is not included in the predefined configuration table yet.

4. The method (100) of claim 1, the method further comprising
determining (S142) if the pairing device (30) stores a driver corresponding to the configuration information of the medical device (10); and
configuring (S142) the pairing device with the driver corresponding to the configuration information of the medical device (10) to receive the broadcast message from the medical device (10) if it is determined that the pairing device (30) stores the driver.

5. The method (100) of claim 4, the method further comprising
determining (S146) if the broadcast message from the medical device (10) can be interpreted based on a predefined translation table stored in the paring device (30); and
generating (SI48) the pairing acknowledge (Ackl) indicating the success of pairing the medical device (10) with the pairing device (30) if it is determined that the broadcast message from the medical device (10) can be interpreted based on the predefined translation table.

6. The method (100) of claim 5, wherein the pairing device (30) stores at least one driver and at least one predefined translation table that are updatable respectively based on an updating information from the guiding device (50) and/or the server (20).

7. The method (100) of any one of claims 1 to 6, wherein the configuration information includes networking settings, the method (100) further comprising:
sending (S210) a networking request (Rst2) to a server (20) of the network based on the networking settings;
waiting (S220) for a networking acknowledge (Ack3) from the server (20) and/or the guiding device (50);
repeating (S230) the sending and waiting steps with a predefined interval within a predefined period before the networking acknowledge (Ack3) is received (S250); and
sending (S260), to the guiding device (50), a notification indicating a success of connecting the medical device (10) to the network if the networking acknowledge (Ack3) is received (S250) from the server or a notification indicating the failure of connecting the medical device (10) to the network if no networking acknowledge (Ack3) is received (S250) from the server (20) or the guiding device (50).

8. A method (100) for connecting a medical device (10) to a network and executed in a portable guiding device (50), the method (100) comprising:
receiving (S110), from a server (20) of the network or from a user interface (54), a pairing request (Rst1);
sending (S120), to a pairing device (30), a pairing message (Msg) including a configuration information for connecting the medical device (10) to the network to enable the pairing device (30) to sense and interpret the broadcast message from the medical device (10);
waiting (S140) for, from the pairing device (30), a pairing acknowledge (Ackl) indicating a success or a failure of pairing the medical device (10) with the pairing device (30); and
sending (S160), to the server (20) or to the user interface (40), a paring notification (Not) indicating the success or the failure of the pairing the medical device (10) with the pairing device (30).

9. The method (100) of claim 5, wherein the configuration information includes at least one of a product model and a wireless communication protocol of the medical device (10).

10. The method (100) of claim 6, wherein the configuration information further includes networking settings, the method (100) further comprising:
receiving (S240), from the server (20), a networking acknowledge (Ack2) indicating that a networking request from the pairing device (30) has been received based on the networking settings by the server (20); and
sending (S250), to the pairing device (30), a networking acknowledge (Ack3) indicating the success of connecting the medical device (10) to the network.

11. A method (100) for connecting a medical device (10) to a network comprising:
receiving (S110), from a server (20) of the network or via a user interface (54), a pairing request (Rst1) by a guiding device (30);
sending (S120), from the guiding device (50) to a pairing device (30) independent from the medical device (10), a pairing message (Msg) including the configuration information for connecting the medical device (10) to the network;
configuring (S140) the pairing device (30) based on the configuration information to enable the pairing device (30) to receive and interpret a broadcast message from the medical device (10) while the guiding device (50) waits for, from the pairing device (30), a pairing acknowledge (Ackl) indicating a success or a failure of pairing the medical device (10) with the pairing device (30);
sending (S150), from the pairing device (30) to the guiding device (50), the pairing acknowledge (Ackl) indicating the success of pairing the medical device (10) with the pairing device (30) if the broadcast message of the medical device (10) can be received and interpreted and the pairing acknowledge (Ackl) indicating the failure of pairing the medical device (10) with the pairing device (30) if the broadcast message of the medical device (10) cannot be received and interpreted; and
sending (S160), from the guiding device (50) to the server (20) or the user interface (54), a pairing notification (Not) indicating the success or the failure of the pairing the medical device (10) with the pairing device (30).

12. A pairing device (30) for connecting a medical device (10) to a network, the pairing device (30) being independent from the medical device (10), the pairing device (30) comprising:
a receiving unit (31) for receiving, from a guiding device (30), a pairing message (Msg) including configuration information for connecting the medical device (10) to the network;
a controlling unit (32) for configuring the pairing device (30) based on the configuration information to enable the pairing device (30) to receive and interpret a broadcast message from the medical device (10); and
a sending unit (33) for sending, to the guiding device (50), a pairing acknowledge (Ackl) indicating a success of pairing the medical device (10) with the pairing device (30) if the broadcast message of the medical device (10) can be received and interpreted.

13. A portable guiding device (50) for connecting a medical device (10) to a network, the portable guiding device (50) comprising:
a receiving unit (51) for receiving a pairing request (Rst1), from a server (20) of the network or from a user interface (54);
a sending unit (53) for sending, to a pairing device (30), a pairing message (Msg) including the configuration information for connecting the medical device (10) to the network to enable the pairing device (30) to sense and interpret the broadcast message from the medical device (10); and
a controlling unit (52) for controlling the receiving unit (51) to wait for, from the pairing device (30), a pairing acknowledge (Ackl) indicating the success or failure of pairing the medical device (10) with the pairing device (30) and controlling the sending unit (53) to send, to the server (20) or to the user interface (40), a paring notification (Not) indicating the success of the pairing the medical device (10) with the pairing device (30).

14. A system (1) for connecting a medical device (10) to a network comprising:
the portable guiding device (50) of claim 13; and
the pairing device (30) of claim 12.

15. A computer readable medium storing a computer program, when executed by a processor or a computer, for performing the method of any one of claims 1-11.
